# EUROPEAN PATENT APPLICATION

(11) **EP 4 621 711 A1**
(43) Date of publication of application: **24.09.2025**
(21) Application number: 24164896.3
(22) Date of filing: 20.03.2024
(51) Int. Cl.: G06T 7/00

(54) **SYSTEM AND METHOD FOR LONGITUDINAL ASSESSMENT OF A BIOMARKER**

(71) Applicant: Siemens Healthineers AG, 91301 Forchheim (DE); Centre Hospitalier Universitaire Vaudois, 1011 Lausanne (CH)
(72) Inventor: Ravano, Veronica, 1024 Ecublens (CH); Kober, Tobias, 1007 Lausanne (CH); Richiardi, Jonas, 1202 Genève (CH)
(74) Representative: Palaci, Ismaël

(57) **Abstract**

The invention relates to a system (200) and method (100) for evaluating a longitudinal evolution of a quantitative biomarker measured for a biological object (210), the method (100) comprising:
- receiving (101), for said biological object (210), longitudinal measurements of said quantitative biomarker, wherein N measurements M_1,...,M_N are received, wherein each measurement M_i, with i = 1,...,N, is performed at a timepoint T_i according to an acquisition technique A_i, wherein if i ≠ j, then T_i ≠ T_j ∀i,j ∈ {1,...,N}, wherein for each measurement M_i, a value V_i is obtained for said biomarker;
- calculating (102), for each measurement M_i, a statistical parameter, which characterizes a variability of the used acquisition technique A_i, said statistical parameter being a measure of a statistical distribution of possible values for the biomarker when carrying out the measurement M_i according to said acquisition technique;
- for each obtained biomarker value V_i, using the calculated statistical parameter for generating (103) synthetic data whose distribution follows the statistical distribution of possible values for the biomarker, wherein each synthetic data represents a simulated value for said biomarker and is assigned the timepoint T_i at which the measurement M_i was performed;
- performing (104) several bootstrapping rounds, wherein, for each bootstrapping, one or several values among the measured value V_i and the biomarker simulated values generated for said measured value V_i are randomly sampled for each timepoint T_i;
- for each bootstrapping, fitting (105) the sampled values by means of a fitting function;
- for each fitting, extracting (106) a fitting parameter of the fitting function;
- evaluating (107) the longitudinal evolution of the quantitative biomarker by statistically comparing the extracted fitting parameters against a reference value.

## Description

### Technical Field

The present disclosure is directed, in general, to methods and systems for characterizing temporal changes of a quantitative biomarker measured for a biological object. More specifically, the present invention is directed to the monitoring of such biomarker by using imaging techniques configured for imaging the biological object, for instance a brain tissue or lesion, said imaging technique being for instance Magnetic Resonance Imaging (MRI).

### Background Art

During radiological viewing, images (e.g., MRI images, x-rays, CTs, etc.) of one time point are often compared to those of another time point in order to track the evolution of a disease. For instance, the effectiveness of radiotherapy treatment is typically assessed by monitoring the tumor size over the duration of the therapy. Similarly, in multiple sclerosis (MS), the evolution of brain lesions is informative of disease activity. Specifically, the presence of "slowly expanding lesions" is a known characteristic of a specific disease progression in MS. Those lesions are characterized by a subtle volumetric expansion over time and their detection by the naked eye is typically challenging as it requires comparing lesion sizes in two different images displayed side by side where the differences might be small. Therefore, methods that provide an estimate of the longitudinal variation (i.e. the temporal variation) of relevant biomarkers over time (e.g., the size and shape of a tumor, of a multiple sclerosis lesion, a volume of a specific organ or brain region, but also for instance the average intensity/texture within a region of interest, or a non-imaging biomarker as blood-based biomarkers, etc.) are needed.

Various solutions have been proposed to improve the monitoring of biomarkers, notably by automating the comparison of biomarkers over time. However, the automatic estimation of a longitudinal evolution of biomarkers suffers from several technical challenges. In fact, the extraction of such biomarkers is sensitive to the variability of the underlying acquisition or measurement technique, as well as the used processing techniques, which reduce the sensitivity to the true, physical change in the tissue of interest. The variability of automatic extraction methods can be caused by technical differences of the input data across different time points (e.g., in MRI, variability of the acquisition protocol and/or the employed hardware or different choice of energy level in CT, different echo angle in ultrasound etc.) which lead to different tissue contrast, signal-to-noise ratio and/or resolution. The resulting variability of the image biomarker extraction algorithms, such as, for example, a lesion segmentation algorithm, can be caused by technical and physical limitations of the acquisition. An example for such a variability-inducing effect is partial volumes, where the intensity of an image voxel is determined by multiple tissue types present inside this voxel (e.g., 70% of a voxel can be composed of lesioned tissue and 30% of healthy tissue); these "mixed voxel" intensities are difficult to classify (e.g., in a segmentation problem "lesion tissue" vs. "healthy tissue") and are hence susceptible to cause also very minor variations between time points.

Therefore, ways to extract longitudinal biomarkers while accounting for the variability of the underlying acquisition and biomarker extraction methods are needed in order to provide a robust estimate of a longitudinal evolution of a biological object and a quantitation of the error engrained in the (longitudinal) measurement.

### Summary of Invention

An objective of the present invention is to propose a method and a system for automatically assessing a longitudinal evolution of a monitored quantitative biomarker that overcome the above-mentioned issues.

Said objective is achieved according to the present invention by a method and a system for automatically quantifying a longitudinal evolution of a quantitative biomarker according to the object of the independent claims. Dependent claims present further advantages of the invention.

More precisely, the present invention concerns a method for quantifying or evaluating the longitudinal evolution of a quantitative biomarker measured for a biological object, the method comprising:
- receiving or acquiring, for said biological object, longitudinal measurements of said quantitative biomarker. For instance, N measurements M_1,..., M_N, performed according to a same or different acquisition techniques typically implemented by one or several acquisition devices known in the art, like an MRI or CT apparatus, are received or acquired. In particular, each measurement M_i is performed at a timepoint T_i according to an acquisition technique A_i, wherein if i ≠ j, then T_i ≠ T_j ∀i,j ∈ {1,...,N}, wherein for each measurement M_i, a value V_i is obtained or measured for said biomarker, wherein N is an integer strictly greater than 1, and i = 1,...,N. Preferentially, the method comprises also performing said longitudinal measurements;
- calculating, for each measurement M_i, at least one statistical parameter P_i (e.g. a variability or reliability coefficient or an error coefficient) which characterizes a variability of the used acquisition technique A_i, said statistical parameter P_i being a measure of a statistical distribution of possible values for the biomarker when carrying out the measurement M_i according to said acquisition technique A_i. Said statistical distribution might be expressed as a confidence interval comprising said possible values that might be taken by the biomarker when carrying out the measurement M_i according to the technique A_i. Said possible values represent potential variations of the measured value V_i that could have been obtained when performing the measurement M_i according to the technique A_i, the statistical distribution showing their likelihood to happen or to be observed. For instance, said statistical parameter might be a variability coefficient that provides an estimation of a variation of the value (e.g. a reliability of the measured value or an error on the measured value, wherein said error comes for instance from the devices used for measuring the biomarker and/or from the method or technique used for extracting the value of said biomarker from the measurement) of the biomarker when carrying out the measurement M_i according to said acquisition technique A_i;
- for each obtained or measured biomarker value V_i, using the calculated statistical parameter P_i for generating synthetic measurement data whose distribution - called hereafter "synthetic distribution" follows said statistical distribution of possible values for the biomarker (i.e. shows a same distribution as said statistical distribution), wherein each synthetic measurement data represents a simulated value for said biomarker and is assigned the timepoint T_i at which measurement M_i giving rise to the value V_i was performed. That means, creating a synthetic distribution of synthetic values whose dispersion is defined by the statistical parameter obtained by the prior step. Typically, said synthetic data are configured for populating an interval of biomarker values comprising (e.g. centered around) said obtained or measured value V_i of the biomarker. Preferentially, the synthetic data distribution is statistically centered around the measured value V_i;
- performing several bootstrapping rounds, wherein, for each bootstrapping, one or several values among the measured value V_i and the biomarker simulated values generated for said measured value V_i are randomly sampled from the synthetic distribution for each timepoint T_i;
- for each bootstrapping, fitting the sampled values by means of a fitting function;
- for each fitting, extracting a fitting parameter of the fitting function;
- evaluating the longitudinal evolution of the quantitative biomarker by statistically comparing the extracted fitting parameters against a reference value, which is notably chosen in function of a null hypothesis and might correspond for instance to a healthy condition or state for said biological object. For instance, said reference value refers to a value that would be obtained for said fitting parameters when considering a null hypothesis;
- preferentially, automatically outputting a result of said evaluation. Preferentially, said result might be displayed on an appropriate display. Said result is typically a representation, e.g. a graph, of said longitudinal evolution of the biomarker, preferentially comprising the reference value for enabling comparison. It is for instance the result of the statistical comparison with the reference value.

The present invention also concerns a system for quantifying or evaluating a longitudinal evolution of a quantitative biomarker measured for a biological object, the system comprising:
- a first interface for receiving or acquiring, for said biological object, longitudinal measurements of said quantitative biomarker, wherein N measurements M_1,..., M_N, are received or acquired, wherein each measurement M_i is performed at a timepoint T_i according to an acquisition technique A_i, wherein if i ≠ j, then T_i ≠ T_j ∀i,j ∈ {1,...,N}, wherein for each measurement M_i, a value V_i is obtained (or measured) for said biomarker, wherein N is an integer strictly greater than 1, and i = 1,...,N. Preferentially, ∀i,j ∈ {1,...,N}, the acquisition technique A_i is the same as the acquisition technique A_j, i.e. the acquisition techniques A_1,...,A_N are one and a same acquisition technique. Alternatively, it exists at least one acquisition technique A_j that is different from the acquisition technique A_i, with i ≠ j and i,j ∈ {1,...,N}.;
- a memory for storing said N measurements and the associated biomarker values;
- a control unit comprising a processor, the control unit being configured for carrying out the steps of the previously described method.

Said system might be used for longitudinal monitoring of said quantitative biomarker. In particular, said system might be part for instance of an MRI or CT apparatus configured for performing the longitudinal measurements and automatically outputting the result of said evaluation according to the previously described method.

### Description of Embodiments

The present invention might be applied to any biomarker that is a measurable quantity typically representing a biological state or condition of a biological object, like a brain. Said biomarker might be for instance:
- a physical characteristic of the biological object or of a region of interest within said biological object, like a volume of said region of interest and/or its size and/or shape;
- a measurable physiological characteristic of said region of interest or of the biological object;
- a measurable biochemical characteristic of said region of interest or of the biological object; or a combination of the latter. For instance, the biomarker might be a lesion volume. In such a case, the acquisition technique may comprise a segmentation of lesions appearing in the biological object. For this purpose, a segmentation algorithm might be used by the system according to the invention for identifying voxels, within the biological object, where a lesion is located. Known in the art techniques for segmenting a biological object might be used. The statistical parameter might be then a measure of the variability of results outputted by the segmentation algorithm. Said statistical parameter can also be calculated by performing test-retest experiments for the acquisition technique A_i used for acquiring the measurement M_i. The estimated variability of each acquisition technique A_i encompasses notably the physical setup used for performing the measurement (e.g., as well as hardware or software resources) and the method, e.g., algorithm, used for extracting the value of the biomarker from a performed measurement. The statistical parameter defines the variability of obtained measured values when repeating said acquisition technique A_i for a same biological object. This enables analyzing changes more robustly that show in the biomarker values over time.

In particular, the present concept works for different acquisition techniques, like MRI acquisition techniques, X-ray acquisition techniques, computed tomography acquisition techniques, ultrasound acquisition techniques, or other acquisition techniques which enable to perform longitudinal measurements of a biomarker, and whose variability might be estimated, using for instance test-retest reliability measurements, or other methods capable of providing an error estimate on extracted or measured values for said biomarker.

The fitting function enables to model the longitudinal changes of the quantitative biomarker while accounting for the reliability of the underlying biomarker acquisition technique, e.g. the extraction technique or method used for obtaining the biomarker value from each measurement. The fitting function might be a linear function, or any appropriate polynomial regression. In particular, statistically comparing the extracted fitting parameters against a reference value may comprise calculating a median estimate of the fitting parameters and testing said median estimate against a null hypothesis. This enables to implement a statistical significance test providing insight on the statistical significance of the obtained result.

Preferentially, said fitting function is configured for fitting a first set of temporally consecutive timepoints independently from a second set of temporally consecutive timepoints, distinct from said first set, in order to independently evaluate, for said first set and said second set, a respective longitudinal sub-evolution of the quantitative biomarker. This provides the advantage of quantifying for instance the effect on the biomarker values of a chemical compound administered to, or a physical action performed on, the biological object that takes place between the first set and second set of timepoints. In particular, said reference value is either a value (e.g., if a null hypothesis is "no change", wherein changes are quantified via the value of a slope extracted from the fitting function, then said reference value would be "0", i.e. "slope = 0"), or a distribution extracted from a set of measurements M'_1,...,M'_K, called reference set, wherein each measurement M'_r, with r=1,...,K and K>1, has been performed at a timepoint T'_r. Said distribution may come from the set of measurements M'_1,...,M'_K performed
(i) for said biological object for which the measurements M_1,...,M_N were performed (called hereafter the tested biological object), wherein the set of measurements M'_1,...,M'_K are acquired at a different time period compared to the measurements M_1,...,M_N, that is the set of consecutive timepoints T'_1,...,T'_K, is different from the set of consecutive timepoints T_1,...,T_N. This can be used for showing an evolution of the biomarker with respect to a pre versus post treatment;
(ii) for another biological object or a group of other biological objects, e.g. healthy biological objects, wherein the set of consecutive timepoints T'_1,...,T'_K, might be the same or different from the set of consecutive timepoints T_1,...,T_N;
(iii) for said tested biological object, but using a different or several different acquisition techniques which serve as reference acquisition technique(s), and which are in the same time period as the time period comprising the timepoints T_1,...,T_N, the set of timepoints T_1,...,T_N being for instance identical to the set of timepoints T'_1,...,T'_K. This enables for instance to compare different acquisition techniques.

In other words, the set of consecutive timepoints T'_1,...,T'_K, might be different from the set of consecutive timepoints T_1,...,T_N, notably when they relate to measurements performed for the same biological object, but also if they relate to a different biological object, or might be the same as, or cover a same period of time as, the consecutive timepoints T_1,...,T_N, notably if they relate to measurements made for a different biological object, or if they relate to measurements made using a reference acquisition technique. The reference set serves as a set of reference for the measurement of the value of the biomarker. For instance, said reference set may comprise measurements performed for a healthy biological object whose longitudinal evolution of the biomarker has to be compared to the longitudinal evolution obtained for the biological object for which the measurement M_1,...,M_N were taken (e.g. if a slope is extracted from the fitting function, then said distribution might represent estimated slope values obtained for a control group of biological objects), or measurements performed for said biological object at a different period of time corresponding to the timepoints T'_1,...,T'_K with respect to T_1,...,T_N. Advantageously, this enables for instance to quantify the evolution of a biomarker before and after a treatment.

### Brief Description of the Drawing

For a more complete understanding of the present disclosure, and the advantages thereof, reference is now made to the following descriptions taken in conjunction with the accompanying drawings, wherein like numbers designate like objects, and in which:
Fig. 1 illustrates a flowchart of a method for evaluating a longitudinal evolution of a quantitative biomarker according to the invention;
Fig. 2 illustrates a system for evaluating a longitudinal evolution of a quantitative biomarker according to the invention;
Fig. 3 shows a graph representing values of a quantitative biomarker in function ot the time;
Fig. 4 illustrates a result obtained via the method according to the invention.

### Description of Examples

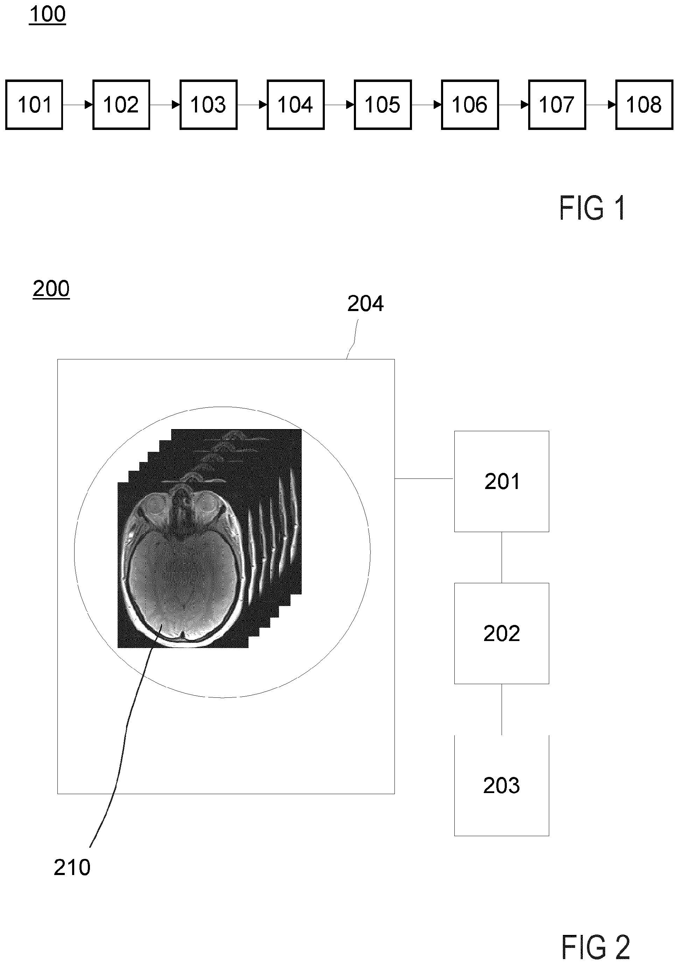

We will now describe in more detail a preferred embodiment of the method according to the invention through Figure 1 and 2, wherein Figure 1 describes the different steps of the method 100 carried out by a preferred embodiment of a system 200 according to the invention which is illustrated by Figure 2.

The preferred embodiments illustrated in Fig. 1 and 2 will be described in the context of lesion monitoring. However, the proposed invention can be applied to any quantitative biomarker. For example, the proposed method can be used to estimate differences in relaxometry measures over time (e.g., T1, T2 relaxation times), or any other biomarker (blood markers, cognitive tests etc.), as long as an estimate of the reliability of cross-sectional measures (i.e. said statistical parameter) can be determined, e.g. via a test-retest setup as illustrated in the preferred embodiments, for generating then said synthetic data.

At step 101, the system 200 acquires or receives N measurements of the quantitative biomarker for a biological object 210. Said N measurements are longitudinal measurements acquired or received via a first interface 201 of the system 200. The latter comprises a control unit 202, connected to said first interface 201, and configured for processing the N measurements. Typically, the acquired or received measurements might be stored in a memory 203 of the system 200 according to the invention. In particular, the system 200 may comprise an imaging apparatus 204 for imaging said biological object 210, for instance a brain as illustrated in Fig. 2. Preferentially, said system 200 is an MRI apparatus configured for acquiring images of the biological object 210, wherein said images comprises voxels whose intensity represents a measurement of said biomarker, and wherein the MRI apparatus is configured for automatically extracting, from said images, a value for said biomarker for the biological object, or for a region of said biological object. According to the present invention, each measurement M_i has been performed at a timepoint T_i that is different from the timepoint at which another measurement has been performed. Each measurement M_i might correspond for instance to an image acquired for said biological object at a timepoint T_i. The acquisition technique enables to extract, for each measurement M_i, a value V_i for the quantitative biomarker, wherein said value V_i has been typically measured for a region of the biological object, or for the whole biological object.

At step 102, the control unit 202 calculates, for each measurement M_i, i.e. for each timepoint T_i, a statistical parameter, e.g. a variability coefficient that is an estimate of a variation of the measured value V_i, when extracting or measuring said value V_i via said acquisition technique. Said statistical parameter is for instance a measurement uncertainty or error estimate that characterizes a statistical dispersion of the measured values V_i that would be obtained for instance when repeating the measurement for the same biological object in the same conditions several times. For example, if the biomarker is a lesion volume that is monitored by acquiring images of the biological object at different timepoints, e.g. by an MRI or CT apparatus, then the value of said volume might be obtained by segmenting the lesion in the acquired images using an existing segmentation algorithm, resulting, for each of said timepoints, to a respective lesion volume estimate. Instead of simply comparing the lesion volume estimates obtained for the different timepoints, the present invention proposes to use a measure of variability of the segmentation algorithm to model synthetic data in a synthetic distribution preferentially centered around the measured value of said lesion volume. This allows to consider the reliability of the segmentation algorithm (which can vary based on differences in processing and imaging techniques) by providing a "certainty" margin around the originally measured lesion volume.

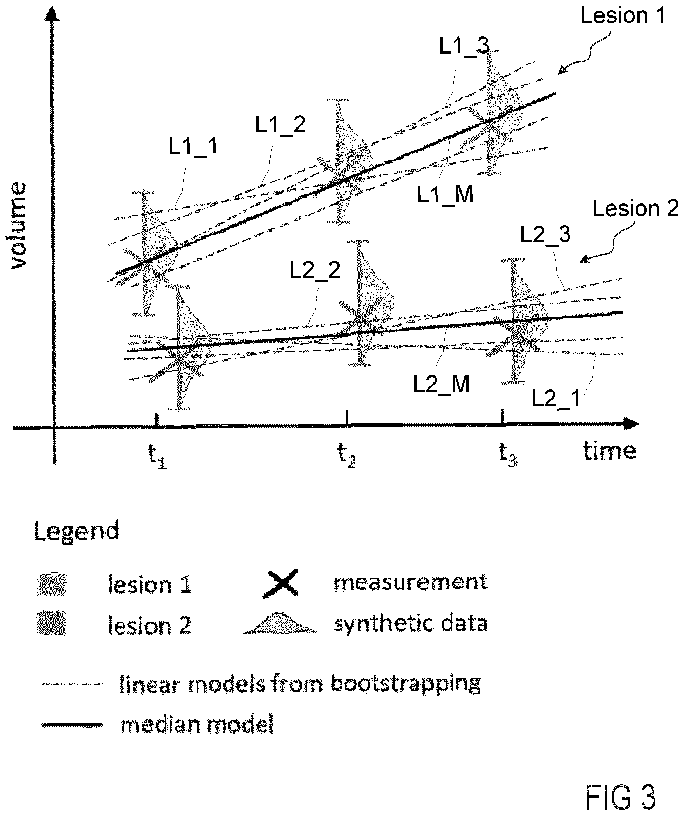

Indeed, at step 103, the control unit 202 uses the calculated statistical parameter for generating said synthetic data. The synthetic data are simulated or calculated data, while said measured value V_i is a real value that has been measured for the biological object. For instance, if the statistical parameter, i.e. the reliability, of the segmentation algorithm estimated in a test retest setting is ±5% and the measured lesion volume is 3 mL, then the control unit 202 might be configured for generating a normal distribution of K synthetic volume lesion values in the interval [3-(5%·3); 3+(5%·3)] mL, which is, in this case, naturally centered around the measured lesion volume, i.e. said 3 mL. This procedure is repeated for all measurements in all available timepoints. This is better illustrated in Figure 3, wherein a first set of measurements is made for a first lesion (Lesion 1) and a second set of measurements is made for a second lesion (Lesion 2), wherein each measurement (represented by a cross x) is made at a timepoint t_i. The extracted biomarker is the volume of the lesion, the graph of Figure 3 showing for each lesion, the evolution of the value of the biomarker (i.e. the lesion volume) in function of the time. For each measurement, synthetic data are generated around the measured lesion volume, wherein the values taken by said synthetic data follow a known in the art distribution, e.g. said normal distribution, around the real value that has been measured, and which is illustrated by a "curve" around the measured value "x" in Fig. 3. In the example of Fig. 3, the synthetic data was generated as a normal distribution centered around the measured lesion volume, and with a width determined by the statistical parameter estimated in a test-retest experiment, giving rise to one hundred synthetic values generated for each measured lesion volume in each time point. While it is proposed here to use a test-retest experiment to estimate the statistical parameter associated to the extraction of the imaging biomarker (lesion volumes), other measures of said statistical parameter, i.e. of the reliability of the biomarker measurements, might also be used. For instance, reliability measures might be estimated by comparing the output of the considered acquisition technique with a ground truth value. In the example of lesions, this could be achieved by measuring an estimation error in lesion volume when extracted using an automated segmentation algorithm using MRI data, with respect to a ground truth physical measure of ex-vivo histopathological samples. Alternatively, in the present context of lesion volume, lesion segmentations could be generated with built-in uncertainty estimates, using for instance Bayesian neural networks, which could then be used for calculating said statistical parameter.

At step 104, the control unit 202 is configured for applying a bootstrapping strategy, wherein, for each timepoint, one or several values among the measured biomarker value and the generated synthetic data for said measured biomarker value are randomly sampled. This means that for each timepoint t_i shown in Fig. 3 for a same biomarker (e.g. for the lesion volume of Lesion 1), one or several values among a set of values comprising the real and the synthetic data obtained for said timepoint are randomly selected. This corresponds to one bootstrapping, and such bootstrapping is performed several times (e.g. P times), resulting thus, for each biomarker, to several (e.g. P) sets of data, wherein each set of data comprises, for each timepoint, one or several randomly selected set values for the biomarker.

At step 105, the control unit 202 is configured for fitting, for each bootstrapping, the sampled values. For example, for each of said several (e.g. P) sets of data, the randomly selected values of the biomarker in function of the time are fitted by a fitting function. As illustrated in Fig. 3, said fitting function might be a linear regression, wherein the different regression lines L1_1, L1_2, L1_3 and L2_1, L2_2 and L2_3 represents respectively for the first lesion and the second lesion different fittings obtained via a linear regression. Several regression lines might thus be obtained for each lesion of Fig. 3, e.g. 100 regression lines, using randomly sampled lesion volumes from the synthetic distributions in each timepoint.

At step 106, the control unit 202 extracts, for each fitting, and thus for each bootstrapping, at least one fitting parameter that will be used for evaluating longitudinal changes of the biomarker. Said fitting parameter can be thus considered as a longitudinal change estimate for the measured biomarker. According to Fig. 3, said fitting parameter is for instance the slope of the different regression lines L1_1, L1_2, L1_3 obtained for the first lesion (Lesion 1), and L2_1, L2_2 and L2_3 obtained for the second lesion (Lesion 2) by fitting the sampled values with a linear regression. The slope of each regression line represents the lesion enlargement (or shrinkage) over time. Instead of a slope, other types of estimate for evaluating longitudinal changes can be defined, including for instance a computation of differences between sampled values, an estimation of a slope from fitting a polynomial line, or any other type of parameter estimated using function fitting (like fitting an exponential).

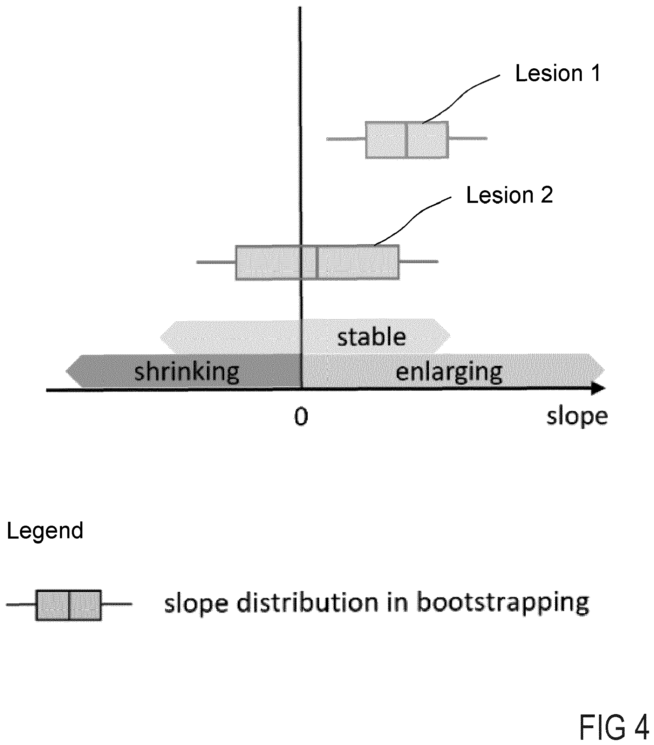

At step 107, the control unit 202 evaluates the longitudinal evolution of the biomarker by comparing the extracted fitting parameters against a reference value. In particular, the control unit 202 may calculate a median value of said at least one fitting parameter. This is illustrated in Fig. 3 by the lines L1_M and L2_M whose respective slope is a median or average value of the slope values of the regression lines L1_1, L1_2, L1_3 and L2_1, L2_2, L2_3 respectively. L1_M represents thus a median model for the longitudinal evolution of the values of the biomarker in function of the time for the first lesion, and L2_M represents another median model for the longitudinal evolution of the values of the biomarker in function of the time for the second lesion. The distribution of the so-extracted fitting parameter(s), e.g. said slopes, is preferentially tested statistically for each lesion, notably against said reference value. For instance, if at least 95% of the slopes extracted from bootstrapping are positive, the lesion is enlarging. Similarly, shrinking and stable lesions (those not showing any significant longitudinal change) might be identified too. This is illustrated in Figure 4, wherein the distribution of the extracted fitting parameters, i.e. the obtained slope distribution for each lesion, is compared to or tested against a reference value assuming no changes, i.e. a Null Hypothesis. This enables to get insight on the statistical significance of the median estimate, i.e. the median value of the slope, characterizing the evolution of the biomarker with time.

At step 108, the control unit 202 may automatically output a result of said evaluation of the longitudinal evolution of the biomarker. For instance, said result might be a graph, as illustrated in Fig. 4, that is automatically displayed, notably on a display of the system according to the invention, or outputted via a second interface of the system 200, which can be the same as the first interface.

The present invention allows to compare the values taken by a biomarker in function of the time, while taking into account the variability of the acquisition or extraction technique used for enabling said longitudinal comparison. The result is a more robust estimate of longitudinal changes of the value of a biomarker as it considers the certainty margin around each cross-sectional measure. Further, the final result is obtained directly for the concerned biological object, and is not based on a comparison with values obtained for a group of similar or identical biological objects. In addition, the present invention enables to associate to each outputted result, i.e. to each evaluation of longitudinal changes of a biomarker a statistical significance level which informs on whether an observed change is worth noting or might be due to technical variabilities. This gives more certainty to the recipient of the respective analysis and might be a great tool for helping said recipient to make a decision.

To summarize, the present invention discloses a system and a method for evaluating a longitudinal evolution of a biomarker, i.e. for quantifying changes of the value of said biomarker over time. It proposes to generate a synthetic distribution of synthetic data to mimic the certainty associated with cross-sectional measurements of said biomarker, to perform bootstrapping in order to generate a pool of estimates of longitudinal biomarker value changes which can then be statistically compared against a reference, e.g. against a null hypothesis, to evaluate or quantify the evolution of the biomarker value with time. In particular, the statistical comparison of the distribution of bootstrapping samples against the null hypothesis may provide a p-value associated with each longitudinal change estimate, e.g. each measured slope, enabling thus to quantify the statistical significance of each longitudinal change estimate.

## Claims

1. Method (100) for evaluating a longitudinal evolution of a quantitative biomarker measured for a biological object (210), the method (100) comprising:
- receiving (101), for said biological object (210), longitudinal measurements of said quantitative biomarker, wherein N measurements M_1,...,M_N, are received, wherein each measurement M_i, with i = 1,...,N, is performed at a timepoint T_i according to an acquisition technique A_i, wherein if i ≠ j, then T_i ≠ T_j ∀i,j ∈ {1,...,N}, wherein for each measurement M_i, a value V_i is obtained for said biomarker;
- calculating (102), for each measurement M_i, at least one statistical parameter which characterizes a variability of the used acquisition technique A_i, said statistical parameter being a measure of a statistical distribution of possible values for the biomarker when carrying out the measurement M_i according to said acquisition technique A_i;
- for each obtained biomarker value V_i, using the calculated statistical parameter for generating (103) synthetic data whose distribution follows the statistical distribution of possible values for the biomarker, wherein each synthetic data represents a simulated value for said biomarker and is assigned the timepoint T_i at which the measurement M_i was performed;
- performing (104) several bootstrapping rounds, wherein, for each bootstrapping, one or several values among the measured value V_i and the biomarker simulated values generated for said measured value V_i are randomly sampled for each timepoint T_i;
- for each bootstrapping, fitting (105) the sampled values by means of a fitting function;
- for each fitting, extracting (106) a fitting parameter of the fitting function;
- evaluating (107) the longitudinal evolution of the quantitative biomarker by statistically comparing the extracted fitting parameters against a reference value.

2. Method (100) according to claim 1, wherein said biomarker is:
- a physical characteristic of the biological object (210) or of a region of interest within said biological object (210);
- a measurable physiological characteristic of said region of interest or of the biological object (210);
- a measurable biochemical characteristic of said region of interest or of the biological object (210).

3. Method (100) according to claim 1 or 2, wherein said biomarker is a lesion volume, said acquisition technique A_i comprises segmenting lesions in the biological object (210) by means of a segmentation algorithm, wherein said statistical parameter is a measure of a variability of results outputted by the segmentation algorithm.

4. Method (100) according to one of the claims 1-3, wherein said statistical parameter is calculated by performing test-retest experiments for said acquisition technique A_i.

5. Method (100) according to one of the claims 1-4, wherein statistically comparing the extracted fitting parameters against a reference value comprises calculating a median estimate of the fitting parameters obtained for said biological object (210) and testing said median estimate against a null hypothesis.

6. Method (100) according to one of the claims 1-5, wherein said fitting function is configured for fitting a first set of consecutive timepoints T_i independently from a second set of consecutive timepoints T_i, distinct from said first set, in order to independently evaluate, for said first set and said second set, a respective longitudinal sub-evolution of the quantitative biomarker.

7. Method (100) according to one of the claims 1-6, wherein said acquisition technique A_i is an MRI acquisition technique or an X-ray acquisition technique, or a computed tomography acquisition technique or an ultrasound-based acquisition technique.

8. Method (100) according to one of the claims 1-7, wherein said fitting function is a linear regression.

9. Method (100) according to one of the claims 1-8, wherein (a) ∀i,j ∈ {1,...,N}, the acquisition technique A_i is the same as the acquisition technique A_j, or wherein (b) it exists at least one acquisition technique A_j that is different from the acquisition technique A_i, with i ≠ j and i,j ∈ {1,...,N}.

10. Method (100) according to one of the claims 1-9, wherein said reference value is either a value, or a distribution extracted from a set of measurements M'_1,...,M'_K, called reference set, wherein each measurement M'_r with r=1,...,K, with K>1, has been performed at a timepoint T'_r, and wherein said measurements M'_1,...,M'_K have been performed for said biological object, or for another biological object.

11. System (200) for evaluating a longitudinal evolution of a quantitative biomarker measured for a biological object (210), the system comprising:
- a first interface (201) for receiving or acquiring, for said biological object, longitudinal measurements of said quantitative biomarker, wherein N measurements M_1,...,M_N, are received or acquired, wherein each measurement M_i is performed at a timepoint T_i according to an acquisition technique A_i, wherein if i ≠ j, then T_i ≠ T_j ∀i,j ∈ {1,...,N}, wherein for each measurement M_i, a value V_i is obtained for said biomarker;
- a memory (203) for storing said N measurements M_i and their respective associated biomarker value V_i;
- a control unit (202) comprising a processor, the control unit being configured for carrying out the steps of the method (100) according to one of the claims 1 to 10.

12. Magnetic Resonance Imaging Apparatus comprising the system (200) according to claim 11, and configured for performing said N measurements.
